# EUROPEAN PATENT APPLICATION

(11) **EP 4 556 552 A1**
(43) Date of publication of application: **21.05.2025**
(21) Application number: 23210136.0
(22) Date of filing: 15.11.2023
(51) Int. Cl.: C12M 1/16, C12M 1/34, C12M 1/36, G01N 21/359

(54) **INTEGRATING ARTIFICIAL INTELLIGENCE IN THE MANUFACTURE OF MYCELIUM-BASED FOOD AND FEED**

(71) Applicant: Millow Holding AB, 421 30 Västra Frölunda (SE)
(72) Inventor: Wainaina, Steven, 50641 Borås (SE); Taherzadeh, Mohammad, 42244 Hisings Backa (SE)

(57) **Abstract**

**Technical field**

The current invention reveals the application of an artificial intelligence aided method whereby near-infrared spectroscopy is employed to model desired parameters. Machine learning is applied to create algorithms from the spectral data to monitor and control process functions during the manufacturing of mycelium-based food and feed products. The mycelium-based food and feed products are obtained through fermentation of non-mushroom filamentous fungi in a high-solid fermentation process characterized by solid loadings exceeding 50 %.

## Description

### Background

As the global population continues to increase, there is a pressing need to increase supply of nutritious food and feed. The agriculture sector is however associated with excess resource depletion and environmental burden which is unsustainable in the long term. In addition to these challenges, there are some health problems that have been linked to meat, the major source of protein globally. There is also an increasing number of consumers who have ethical reservations regarding how animals intended for consumption are treated. Although plant-based protein sources may be more a sustainable option and ethically acceptable compared to their animal-based counterparts, there are some drawbacks associated with such sources. A major drawback is the presence of antinutritional compounds, such as phytate, which can interfere with the absorption of nutrients during digestion. Furthermore, soybeans which contribute the largest share of plant-based proteins have recently attracted criticism due to the destruction of rainforests to create more land space for their cultivation. As a consequence of the said activities, there is a high risk of eco-diversity loss, as well as decline in natural carbon sinks which are contrary to the vision captured in the global sustainability goals.

In light of the above, there is an urgent need to develop sustainable technologies that can provide alternative healthy protein-rich diets. Filamentous fungi, particularly the non-mushroom types, can be utilized as substitutes for production of protein-rich food products. The fungi can be cultivated on a variety of plant-based materials in which the fermented products have better nutrition quality, bioaccessibility, and digestibility compared to the non-fermented materials. Furthermore, the fungi can synthesize vitamins, and alter the protein and lipid profile of the fermented substrate due to the fungal metabolic activities. Fungi can also contribute to the supply of functional food products with less environmental impact and requiring considerably less resources such as water and land compared to animal-based food production. Filamentous fungi can also be co-fermented with other microorganisms, including bacteria, to impart important characteristics in the final products such as presence of probiotics.

There are two principle methods to cultivate filamentous fungi, i.e. using submerged or solid-state fermentation (SSF) techniques. Submerged fermentation has been well developed and commercialized for fungi-based products. Quorn^{™} products made using *Fusarium venenatum* are a classic example of implementation of submerged fermentation. On the other hand, solid-state fermentation has been used for centuries in Asia for traditional foods and other key intermediates applied in the food industry. Examples include tempeh, which is made by fermenting soybeans using *Rhizopus oligosporus,* and koji, which is made using *Aspergillus oryzae.* The existing SSF processes are however characterized by long fermentation time and labor-intensive manual operations. Such characteristics limit the suitability of the SSF technology for current modern-day food production processes that require speed, accuracy, high levels of safety standards and flexibility. Furthermore, SSF technology requires close control due to the heat and mass transfer issues that can hinder optimum heat dissipation, affecting the quality of fungal biomass production. In addition, conventional SSF process involve laborious and time-consuming quality control (QC) procedures. After the inoculation procedure, the inoculated substrate is transferred to a fermentation device where the appropriate conditions, mainly the aeration rate, temperature, and relative humidity, are applied to initiate and maintain fungal growth. The fermentation process in high-solid processes proceeds for a period of several days, in some cases up to 14 days, in the prior art. After the fermented material is discharged from the fermentation devices, it undergoes a series of off-line QC procedures either prior to or within the downstream processes. These QC operations are extensively manual in nature, requiring additional time scheduling during process design.

Artificial intelligence (AI) is a promising tool for coordinating complex manufacturing processes. The benefits of utilizing AI in manufacturing processes include improving the product quality, maximizing process safety, enhancing productivity, minimizing downtime losses, enhancing production yields, reducing the overall cost, and ultimately increasing business profitability. AI techniques are built upon machine learning algorithms, for instance, artificial neural network (ANN), logic programming, reinforcement learning, expert system, fuzzy logic (FL), and swarm intelligence which are useful in process estimation and decision making. In addition to machine learning algorithms, AI-based decision-making systems employ tools such as laser-technology-based systems, X-ray-based systems electronic nose (E-nose), electronic tongue (E-tongue), high-resolution cameras and near-infrared (NIR) spectroscopy. NIR technology, in particular, operates on the known principle of molecular vibrations associated with the X-H bond type, such as O-H, N-H, C-H and S-H, and their connection with infrared radiation. The spectral range covered by NIR is between 700 - 2500 nm. As a result of weak absorption of NIR energy, direct measurement of solid samples that are strongly absorbing and highly light scattering can be achieved in the reflection measurement mode. In-line and on-line analyses are readily implementable with NIR thereby facilitating continuous quality control and process monitoring. Adequate calibration to apply the analytical data in the NIR spectra can be performed using known techniques that rely on chemometric and/or machine learning principles.

Several examples of the application of AI in filamentous fungi-based bioprocesses are provided in literature (Wainaina and Taherzadeh, Bioresource Technology, 2023, 369, 128421). For instance, during cultivation of *Trichoderma reesei,* a soft sensor relying on Multiphase Artificial Neural Network (MANN) was developed to predict the biomass concentration in submerged fermentation (Murugan and Natarajan, Journal of Microbiological Methods, 2019, 159, p. 5-11). In another study, the analysis of the aqueous fraction containing enzymes produced by *R. oligosporus* has also been practiced with the help of ANN models built using NIR spectral data using SSF (Barchi et al., Process Biochemistry, 2016, 51 (10), p. 1338-1347). In the food industry, AI technologies have been used in the beverage sector such as during prediction of the fermentation process in ethanol production (Itto-Nakama, K., et al., Bioscience, Biotechnology, and Biochemistry, 2022, 86 (1), p. 125-134), during the quality assessment of beer (Gonzalez Viejo and Fuentes, Fermentation, 2020, 6, 104) and in the production system of black tea (CN103749745B). Furthermore, NIR spectroscopy has been employed for identification, grading, recognition, classification, sorting and non-destructive evaluation of mushrooms as revealed in previous patent filings (CN109447045A, CN103008249A, CN103177257A, CN207076673U, CN108226148A, CN108875913A).

Food and feed products can be manufactured using non-mushroom filamentous fungi in a resource efficient SSF process with high solid-loadings exceeding 50%. Such high solid loading complicates the mass transfer of oxygen to the fungal cells and derails the fungal growth thereof. Furthermore, maintaining optimum mycelium propagation, intensity and overall quality is a difficult task given the existing state of the art off-line monitoring and control methods. Some of the activities on the upstream side of the said fungi-based manufacturing process include inoculum preparation and substrate conditioning. The viability and sterility of the seed inoculum are extremely critical as they determine the success of all the subsequent manufacturing stages. It is required then, that appropriate control mechanisms are put in place to guarantee the highest inoculum quality. On the other hand, the substrate condition is determined by the desired final product characteristics. The properties to be considered include particle size, shape, and water activity. The disinfection of the substrate is, however, not optional. Mixing the inoculum and substrate should consider the inoculum size, moisture content, porosity, mixing speed and intensity. Factors such as pre-forming, weight, and bed height are also essential prior to initiating the fermentation process. The main fermentation stage is mainly influenced by aeration rate, temperature, and relative humidity. Regarding the downstream operations, quality control is needed to check moisture content, the density and orientation of fungal filaments before and after post-forming as well as heat treatment steps.

Regarding monitoring of SSF processes aimed at production of food or feed using non-mushroom fungi, some techniques have been developed and applied. For instance, off-line analysis of CO₂ to monitor the growth of *Neurospora intermedia* on stale bread in an SSF process operated in semi-continuous mode has been performed (Wang, R., et al., Biochemical Engineering Journal, 2021, 169, 107959). The generated data was processed in Matlab to model the CO₂ evolution rate. In another study, a non-destructive method to monitor the SSF of barley usingR. *oligosporus* and lactic acid bacteria (LAB) was developed (Feng X.M., et al., Journal of Applied Microbiology, 2007, 103(4), p. 1113-1121). The process involved a high-resolution digital camera while image analysis was processed via an algorithm optimized to the changes in images of the fermented products with reference to the visible barley grains. A similar study was performed in which colony color was the basis of evaluating fungal growth during SSF of *Rhizopus oryzae* on wheat bran and wheat stillage (López-Gómez, J.P., et al., Journal of Microbiological Methods, 2019, 160, p. 60-67). General Image Manipulation Program (GIMP) software was utilized for analysis of colony occupation areas and measurement of color intensity in the images captured using a NIKON D3200 camera.

However, the application of NIR and machine learning to monitor and control food and feed production systems based on non-mushroom filamentous fungi produced in high-solid loading systems has not been implemented in prior art. The present invention thus reveals the development of a reliable AI method for on-line and at-line monitoring and control of the said manufacturing processes. The invention relates to manufacturing processes that are only aimed at the production of whole fungal biomass, and not the production of metabolites or biochemicals from the non-mushroom fungi such as the production of organic acids, alcohols, enzymes, aroma compounds, and pigments.

### Summary of invention

In view of the above outlined issues, the present invention developed an intelligent quality control system in the entire food and feed manufacturing line utilizing non-mushroom filamentous fungi at high organic loadings of between above 50%. Unlike the mushroom-forming fungi that are known to have a fruiting body (constituting the main edible portion) in their later stages of development, the filamentous fungi referred to in this invention do not form a fruiting body. The specific edible filamentous fungi covered in this invention are *Aspergillus oryzae, Neurospora intermedia, Rhizopus oligosporus,* and *Rhizopus oryzae.* These fungi propagate by means of filaments or mycelium entwined to form a matrix structure together with part of the substrate in the fermentation system.

At the core of the present invention is the establishment of an AI guided method that guarantees production of high quality food and feed fungi-based products. Achieving the desired final product quality is a function of several factors including the physicochemical condition of the substrates, the inoculation procedure, intensity and method of aeration, temperature gradients and the degree of relative humidity. Compared to prior art, obtaining the optimal final product quality at the high solid loadings is a result of an extremely complex balance of all the mentioned factors.

In order to reach the above objectives, a system according to one of the aspects of the present invention includes devices and methods for NIR data capture, processing, and automatic process control. The NIR data capture system receives an input, that is the generated spectral data at the described stages within the manufacturing line. The processing unit utilizes the input data from the NIR data capture system and converts it to a trained AI algorithm. The processing unit triggers the AI to compute the desired characteristics at the intended manufacturing stages. The data processing system outputs the estimated data computed by AI and compares with the reference data for subsequent process control. Transmission of data is preferably done wirelessly. The process control entails remote triggering of the actuators that induce the intended properties on the fermentation materials during the manufacturing stage in focus.

One aspect of the present invention has its object to facilitate rapid detection of flaws or defects that may take place during substrate preparation, inoculation, mixing, fermentation and in the downstream stages during the final product preparation. According to the method presented in this invention, data generated prior to the initiation of the actual manufacturing process is utilized to train a model. Calibration is performed using machine learning which subsequently employs the monitored data in real time by use of NIR technology. Algorithms are created using the NIR spectral data to define the desired physicochemical properties along the distinct stages of the manufacturing line. Discovery of any deviations from the ideal is acted upon in a timely manner to avoid loss of production batches, evading process downtime, minimizing product loss while attaining highest productivity levels.

An object of another aspect in the present invention is to gather real-time NIR spectral data at specially designated locations along the manufacturing line. The spectral data is collected with the help of high-resolution cameras where the reflected light is evaluated through multiple wavelengths to generate a spectral profile.

In one aspect of the current invention, the present inventors developed an effective method for automated operation of the upstream side of the manufacturing line, that is the inoculum preparation and substrate conditioning, that must be maintained to the highest possible standards. For the inoculum, the main aim is to identify utilization of highly viable cells. The inoculum is understood within the context of the present invention as biomass manufactured using non-mushroom filamentous fungi at high organic loadings exceeding 50% and grown primarily on oat, wheat or rye grains, or the mixtures thereof. During the mixing of the ingredients to be subjected to fermentation in the succeeding steps, maximum homogeneity and porosity is targeted for smart optimization according to the data gathered by the at least one NIR scanner in the mixer.

In another aspect of this invention, the fermentation step is achieved through provision of the correct fermentation parameter that is demanded at the different growth phases. Furthermore, the fermentation system comprises a computing device configured to identify the fungal growth state from the data measured by the at least one NIR scanner. The computing device may host the spectral image recognition system. It uses machine learning algorithms to detect fully ready fungal biomass based on the intensity of the mycelial filaments onto the supplied substrate. Alternatively or in combination, it may also detect the ready fungal biomass directly based on the surface characteristics defined by the spectral image.

### Description of the invention

The present invention relates to an artificial intelligent (AI) assisted manufacturing edible products based on high-solid fermentation using non-mushroom filamentous fungi. More particularly, machine learning is applied in the upstream, fermentation and downstream stages to monitor and control the quality of the inoculum, the ratios of all ingredients prior to mixing, and the quality of the resulting filamentous fungal biomass entering the post-treatment stages. The invention provides a solution to guaranteeing an optimum recipe of ingredients for fermentation is reached which is kept consistent during every cycle. One of the main benefits of the present invention is enhancing process efficiency by minimizing the time required for upstream stages, the actual fermentation, and the post-processing stages of the fermented food. The other key benefit is providing an automated and rapid control mechanism that increases the safety and raises the quality of the final food or feed product to premium classification compared to the existing manufacturing techniques.

The purpose of the present invention is to solve the problems associated with the above-described non-mushroom fungi manufacturing processes for food and feed with a solid loading of above 50% using AI techniques. These techniques are built upon machine learning which relies on a number of algorithms and high-resolution NIR cameras. The ANN architecture defines how the artificial neurons are connected to one another and is trained to predict the output variables. Optimization of the weights and biases from the input data obtained using the NIR spectrometer is performed to reduce the squared error between the calculated value and the observed value. This strategy results in an approximate output value and the training is terminated when according to pre-established benchmark, the neural network reaches a generalized result for multiple problems. The present invention applies Levenberg-Marquardt calibration method and can be implemented using e.g. Phython. For the training and test tests of the models, the independent experimental models were randomly divided and ran in triplicate, with 80% used for training and 20% for tests.

The several approaches outlined above may be coded as software that is executable on one or more computer systems that utilize any one of an assortment of platforms or operating systems. In addition, such software may be built using any of a number of appropriate programming languages and/or programming or scripting tools. The software may be assembled as executable machine language code or intermediate code that is implemented on a framework or virtual machine.

Based on the above, the present invention provides a system for controlling and monitoring the production process of food and feed based on edible non-mushroom filamentous fungi produced at high solid loading fermentation conditions. The said methods are applied as described in the following example. For examples 1 - 6, the NIR detection is performed at-line, while examples 7 - 20, the NIR detection and subsequent adjustment of process parameters is performed online.

### Example 1

The measurements were carried out with the contact of MicroNIR device and sporulated matter to be used as inoculum containing the starter edible fungal spores of *Asperillus oryzae.* MicroNIR spectrometer was used to analyze the external surface of 10 different locations. The measurement distance between the scanning camera and the sporulated matter was kept at a distance of 10 mm.

The physical dimensions of the sporulated matter sample were approximately (L x W x H) 50 mm by 50 mm by 20 mm. The spectral data averaged from 50 scans, was recorded as the logarithm of the inverse of an exposure time of 32 ms, set a translation stage moving speed of 1.2 cm/s at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the coloration of the sporulated material. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the succeeding production step, i.e. mixing the *Asperillus oryzae* inoculum with the selected substrate, accordingly by adjusting the inoculum to substrate ratio.

### Example 2

The measurements were carried out with the contact of MicroNIR device and sporulated matter to be used as inoculum containing the starter edible fungal spores of *Rhizopus oligosporus.* MicroNIR spectrometer was used to analyze the external surface of 10 different locations. The measurement distance between the scanning camera and the sporulated matter was kept at a distance of 10 mm.

The physical dimensions of the sporulated matter sample were approximately (L x W x H) 50 mm by 50 mm by 20 mm. The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the coloration of the sporulated material. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the succeeding production step, i.e. mixing the *R*. *oligosporus* inoculum with the selected substrate, accordingly by adjusting the inoculum to substrate ratio.

### Example 3

The measurements were carried out with the contact of MicroNIR device and sporulated matter to be used as inoculum containing the starter edible fungal spores of *R. oryzae.* MicroNIR spectrometer was used to analyze the external surface of 10 different locations. The measurement distance between the scanning camera and the sporulated matter was kept at a distance of 10 mm.

The physical dimensions of the sporulated matter sample were approximately (L x W x H) 50 mm by 50 mm by 20 mm. The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the coloration of the sporulated material. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the succeeding production step, i.e. mixing the *R. oryzae* inoculum with the selected substrate, accordingly by adjusting the inoculum to substrate ratio.

### Example 4

The measurements were carried out with the contact of MicroNIR device and oat grains to be used as substrate. MicroNIR spectrometer was used to analyse the external surface of 10 - 20 different locations, preferably 15 locations. The measurement distance between the scanning camera and the substrate was kept at a distance of 10 mm. The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the succeeding production step, i.e. mixing the oat grains with the selected inoculum, accordingly by adjusting the inoculum to substrate ratio.

### Example 5

The measurements were carried out with the contact of MicroNIR device and wheat grains to be used as substrate. MicroNIR spectrometer was used to analyse the external surface of 10 - 20 different locations, preferably 15 locations. The measurement distance between the scanning camera and the substrate was kept at a distance of 10 mm. The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the succeeding production step, i.e. mixing the wheat grains with the selected inoculum, accordingly by adjusting the inoculum to substrate ratio.

### Example 6

The measurements were carried out with the contact of MicroNIR device and rye grains to be used as substrate. MicroNIR spectrometer was used to analyse the external surface of 10 - 20 different locations, preferably 15 locations. The measurement distance between the scanning camera and the substrate was kept at a distance of 10 mm. The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the succeeding production step, i.e. mixing the rye grains with the selected inoculum, accordingly by adjusting the inoculum to substrate ratio.

### Example 7

The measurements were carried out with the contact of NIR capturing devices attached to a frame bolted to the inside chamber of the mixer. Initially, water (18% w/w), oats (72% w/w), and *Aspergillus oryzae* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *Aspergillus oryzae.* The measurement distance between the scanning camera and the mixture was kept at a distance of 10 mm.

The spectral data averaged from 50 scans, was recorded at 1-nm intervals from 900 to 1700 nm.The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data was sent to the integrated central command center which through a feedback loop aligns the aimed physicochemical properties of water activity and porosity in the mixer accordingly.

### Example 8

The measurements were carried out with the contact of NIR capturing devices attached to a frame bolted to the inside chamber of the mixer. Initially, water (18% w/w), wheat grains (72% w/w), and *Aspergillus oryzae* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *Aspergillus oryzae.* The measurement distance between the scanning camera and the mixture was kept at a distance of 10 mm.

The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data was sent to the integrated central command center which through a feedback loop aligns the aimed physicochemical properties of water activity and porosity in the mixer accordingly.

### Example 9

The measurements were carried out with the contact of NIR capturing devices attached to a frame bolted to the inside chamber of the mixer. Initially, water (18% w/w), rye (72% w/w), and *Aspergillus oryzae* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *Aspergillus oryzae.* The measurement distance between the scanning camera and the mixture was kept at a distance of 10 mm.

The spectral data averaged from 50 scans, was recorded at 1-nm intervals from 900 to 1700 nm. The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data was sent to the integrated central command center which through a feedback loop aligns the aimed physicochemical properties of water activity and porosity in the mixer accordingly.

### Example 10

The measurements were carried out with the contact of NIR capturing devices attached to a frame bolted to the inside chamber of the mixer. Initially, water (18% w/w), oats (72% w/w), and *R. oryzae* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyze the mixed material consisting of water, oats and *R. oryzae.* The measurement distance between the scanning camera and the mixture was kept at a distance of 10 mm.

The spectral data averaged from 50 scans, was recorded at 1-nm intervals from 900 to 1700 nm.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 11

The measurements were carried out with the contact of NIR capturing devices attached to a frame bolted to the inside chamber of the mixer. Initially, water (18% w/w), wheat grains (72% w/w), and *R. oryzae* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyze the mixed material consisting of water, oats and *R. oryzae.* The measurement distance between the scanning camera and the mixture was kept at a distance of 10 mm.

The spectral data averaged from 50 scans, was recorded at 1-nm intervals from 900 to 1700 nm.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 12

The measurements were carried out with the contact of NIR capturing devices attached to a frame bolted to the inside chamber of the mixer. Initially, water (18% w/w), rye (72% w/w), and *R. oryzae* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *R*. *oryzae.* The measurement distance between the scanning camera and the mixture was kept at a distance of 10 mm.

The spectral data averaged from 50 scans, was recorded at 1-nm intervals from 900 to 1700 nm.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 13

The measurements were carried out with the contact of NIR capturing devices attached to a frame bolted to the inside chamber of the mixer. Initially, water (18% w/w), oats (72% w/w), and *Rhizopus oligosporus* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *Rhizopus oligosporus.* The measurement distance between the scanning camera and the mixture was kept at a distance of 10 mm.

The spectral data averaged from 50 scans, was recorded at 1-nm intervals from 900 to 1700 nm.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 14

The measurements were carried out with the contact of NIR capturing devices attached to a frame bolted to the inside chamber of the mixer. Initially, water (18% w/w), wheat grains (72% w/w), and *Rhizopus oligosporus* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *R*. *oligosporus.* The measurement distance between the scanning camera and the mixture was kept at a distance of 10 mm.

The spectral data averaged from 50 scans, was recorded at 1-nm intervals from 900 to 1700 nm.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 15

The measurements were carried out with the contact of NIR capturing devices attached to a frame bolted to the inside chamber of the mixer. Initially, water (18% w/w), rye (70% w/w), coriander (2% w/w) and *Rhizopus oligosporus* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *R. oligosporus.* The measurement distance between the scanning camera and the mixture was kept at a distance of 10 mm.

The spectral data averaged from 50 scans, was recorded at 1-nm intervals from 900 to 1700 nm.The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the moisture content of the substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 16

The measurements were carried out with the contact of NIR capturing devices attached to a frame bolted to the inside chamber of the fermentation device. Fermenting material containing initial amounts of water (15% w/w), oats (65%w/w), white onions (5% w/w) and *Rhizopus oligosporus* (15%w/w) are placed on the fermentor's bed surface. The fermentation conditions were initially set as relative humidity of 75%, temperature of 30 °C, air flow rate of 0.4 vvm (volume per volume per minute), and pH of 5.0 .

The NIR spectrometer was used to analyze the mixed material consisting of water, oats and *R. oligosporus.* The measurement distance between the scanning camera and the mixture was kept at a distance of 10 mm every 20 min. The spectral data averaged from 50 scans, was recorded at 1-nm intervals from 900 to 1700 nm. The light source was set at an angle of 90° to the horizontal direction of the sample. After every 4 hours, the supply of the process parameters described above, i.e. relative humidity, temperature, and air flow rate are paused for 5 min.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the fermented product into (i) well fermented level, (ii) averagely fermented level, and (iii) reject based on the remaining starch content of the substrate or on the level of sporulation. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 17

The measurements were carried out with the contact of NIR capturing devices attached to a frame bolted to the inside chamber of the mixer. Initially, water (18% w/w), wheat grains (72% w/w), and *Neurospora intermedia* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyse the mixed material consisting of water, oats and *N. intermedia.* The measurement distance between the scanning camera and the mixture was kept at a distance of 10 mm.

The spectral data averaged from 50 scans, was recorded at 1-nm intervals from 900 to 1700 nm.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the remaining starch content. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 18

The measurements were carried out with the contact of NIR capturing devices attached to a frame bolted to the inside chamber of the mixer. Initially, water (18% w/w), rye (70% w/w), coriander (2% w/w) and *Neurospora intermedia* (10% w/w) are added to the mixer. The rotation of the mixer was set to 100 rpm. Within intervals of 5 - 15 minutes, preferably 10 minutes, the mixer rotation is temporarily paused. During the pause, the NIR spectrometer was used to analyze the mixed material consisting of water, oats and *N. intermedia.* The measurement distance between the scanning camera and the mixture was kept at a distance of 10 mm.

The spectral data averaged from 50 scans, was recorded at 1-nm intervals from 900 to 1700 nm.The light source was set at an angle of 90° to the horizontal direction of the sample.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the samples into (i) most acceptable level, (ii) average acceptable level, and (iii) reject based on the remaining starch content. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 19

The measurements were carried out with the contact of NIR capturing devices attached to a frame bolted to the inside chamber of the fermentation device. Fermenting material containing initial amounts of water (15% w/w), oats (65%w/w), white onions (5% w/w) and *Aspergillus oryzae* (15%w/w) are placed on the fermentor's bed surface. The fermentation conditions were initially set as relative humidity of 75%, temperature of 30 °C, air flow rate of 0.4 vvm (volume per volume per minute), and pH of 5.0 .

The NIR spectrometer was used to analyze the mixed material consisting of water, oats and *Aspergillus oryzae.* The measurement distance between the scanning camera and the mixture was kept at a distance of 10 mm every 20 min. The spectral data averaged from 50 scans, was recorded at 1-nm intervals from 900 to 1700 nm. The light source was set at an angle of 90° to the horizontal direction of the sample. After every 4 hours, the supply of the process parameters described above, i.e. relative humidity, temperature, and air flow rate are paused for 5 min.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the fermented product into (i) well fermented level, (ii) averagely fermented level, and (iii) reject based on either the remaining starch content or on the level of sporulation. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

The data is sent to the integrated central command center which through a feedback loop aligns the aimed physicochemical properties of water activity and porosity in the mixer accordingly.

### Example 20

The measurements were carried out with the contact of NIR capturing devices attached to a frame bolted to outside the chamber of the fermentation device. Fermented material containing initial amounts of water (15% w/w), oats (65%w/w), white onions (5% w/w) and *Rhizopus oligosporus* (15%w/w)

The NIR spectrometer was used to analyze the said fermented material. The measurement distance between the scanning camera and the mixture was kept at a distance of 10 mm. The spectral data averaged from 50 scans, was recorded at 1-nm intervals from 900 to 1700 nm. The light source was set at an angle of 90° to the horizontal direction of the fermented sample. After every 4 hours, the supply of the process parameters described above, i.e. relative humidity, temperature, and air flow rate are paused for 5 min.

A material with the highest diffuse reflectance, Spectralon^{®}, was used as the white reference prior to the NIR spectral analysis. The reference spectral signal is subtracted from the sample spectral signal to eradicate the background noise interference. Building a back propagation optimization algorithm is done using Levenberg-Marquardt method. Furthermore, the artificial neural network estimator based on the Levenberg-Marquard algorithm employs a fusion of Steepest Descent method and Gauss-Newton.

Models were built using the raw values of NIR absorbance in the entire spectral range as inputs to classify the fermented product into (i) acceptable level, (ii) averagely acceptable level, and (iii) reject based on regarding the visual appeal, density, extent of sporulation, orientation and intensity of fungal filaments and fraction of remaining substrate. The spectral data were divided randomly as 80% of the samples used for training and 20% for testing the model. To assess the model performance, a neuron trimming test was performed to select the model with no underfitting or overfitting. Normalizing input and output data for the ANN models was done to avoid huge variance in the magnitude between the weights and distances, which can lead to unreal patterns thus compromising the model.

Those skilled in the art will readily observe that numerous modifications and alterations of the methods described herein may be made while retaining the teachings of the invention.

### Figures

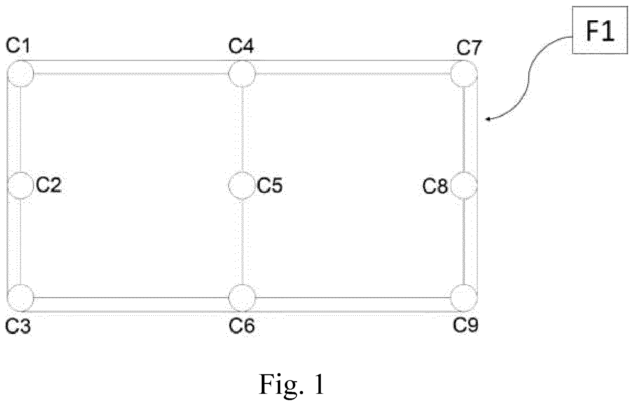

Fig. 1. NIR camera mounting frame used in the mixer or fermentation device
Fig 1. NIR scans of samples that are used to train the detection model for fermented products

Fig. 1 shows the NIR capturing devices (C1-C9) attached to a frame (F1) bolted to the inside chamber of the mixer or fermentation device.

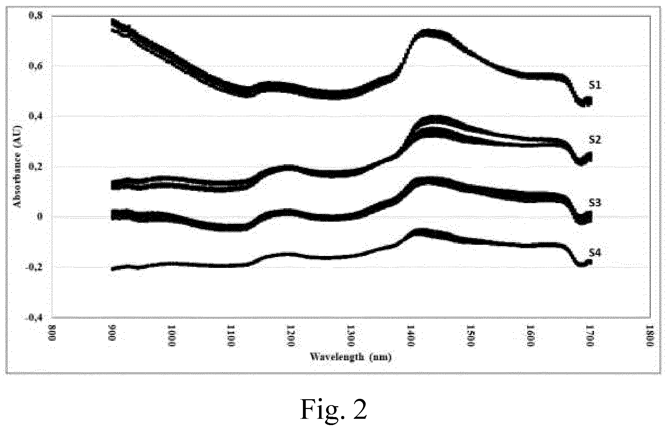

Fig. 2 shows a typical NIR spectral data obtained from the fermentor. S1 represents the reject based on the sporulation level, S2 represents well fermented level, S2 represents averagely fermented level, and S4 represents the reject based on the remaining starch content.

## Claims

1. An artificial intelligence (AI) aided method based on near infrared (NIR) spectrometry to manufacture filamentous fungi-based products in a fermentation process **characterized by** a solid loading of above 50%;
a) wherein the edible filamentous fungi comprise the non-mushroom strains, consisting of *Neurospora intermedia, Rhizopus oligosporus, Aspergillus oryzae* or *Rhizopus oryzae* or a mixture thereof;
b) wherein NIR sensors are incorporated in the mixing and fermentation devices for at-line and on-line detection respectively;
c) wherein Artificial Neural Networks (ANN) are used to develop models used to predict the performance of the mixing and fermentation steps;
d) wherein a feedback mechanism is established to prescribe corrective measures on identified deviation from the defined accepted patterns.
e) wherein the inoculum is backsloped from a previous fermentation cycle;
f) wherein the main substrate is a grain consisting of oat, wheat, rye or a mixture thereof;
g) wherein the minor ingredient is a vegetable or fresh spice, such as onions and coriander.

2. An AI aided method of claim 1, wherein ANN is applied to detect the quality of the inoculum used for seeding the subsequent fermentation batches prior to mixing with the fresh substrate.

3. An AI aided method of claim 1, wherein ANN is applied to determine and control the ratios of the inoculum, main substrate, minor ingredients and water activity during the mixing step prior to fermentation.

4. An AI aided method of claim 1, wherein ANN is applied to detect the quality of the fungal biomass obtained after fermentation regarding the visual appeal, density, extent of sporulation, orientation and intensity of fungal filaments and fraction of remaining substrate.

5. An AI aided method of claim 1, wherein ANN is applied to grade the filamentous fungal biomass from the fermentation step and to accept or reject for the subsequent post-processing steps.

6. A food product made from the accepted filamentous fungal biomass in claim 5.

7. A feed product made from the accepted filamentous fungal biomass in claim 5.
